Europäisches Patentamt

(19) European Patent Office

Office européen des brevets

(11) Veröffentlichungsnummer: **0 185 331**
**B1**

(12) **EUROPÄISCHE PATENTSCHRIFT**

(45) Veröffentlichungstag der Patentschrift:
13.09.89

(21) Anmeldenummer: **85115942.6**

(22) Anmeldetag: **13.12.85**

(51) Int. Cl.⁴: **A 61 K 31/52,** A 61 K 9/52

(54) **Theophyllin-Retardzubereitung.**

(30) Priorität: **21.12.84 CH 6111/84**
     **24.06.85 CH 2683/85**

(43) Veröffentlichungstag der Anmeldung:
    **25.06.86 Patentblatt 86/26**

(45) Bekanntmachung des Hinweises auf die Patenterteilung:
    **13.09.89 Patentblatt 89/37**

(84) Benannte Vertragsstaaten:
    **AT BE CH DE FR GB IT LI LU NL SE**

(56) Entgegenhaltungen:
    **EP-A-0 122 077**
    **WO-A-83/00284**
    **FR-A-2 237 620**
    **GB-A-857 550**

    **JOURNAL OF PHARMACEUTICAL SCIENCES,**
    **Band 72, Nr. 7, Juli 1983, Seiten 772-775, American**
    **Pharmaceutical Association, Washington, US; G.**
    **KÄLLSTRAND et al.: "Membrane-coated tablets: a**
    **system for the controlled release of drugs"**
    **CHEMICAL ABSTRACTS, Band 96, Nr. 1, Januar**
    **1982, Seite 336, Nr. 11678k, Columbus, Ohio, US; &**
    **JP - A - 81 122 311**

(73) Patentinhaber: **Byk Gulden Lomberg Chemische**
    **Fabrik GmbH, Byk- Gulden- Strasse 2, D-7750**
    **Konstanz (DE)**

(72) Erfinder: **Benedikt, Gerald, Dr., Bradenburger**
    **Strasse 1, D-7750 Konstanz (DE)**
    Erfinder: **Steinijans, Volker, Dr., Jakobstrasse 32,**
    **D-7750 Konstanz (DE)**

**EP 0 185 331 B1**

**Beschreibung**

Die Erfindung betrifft eine Theophyllin-Retardzubereitung.

Theophyllin (1,3-Dimethylxanthin) ist ein altbewährter Arzneistoff zur Therapie der obstruktiven Atemwegserkrankungen. Aufgrund seiner interindividuell stark differierenden Pharmakokinetik und des sehr engen therapeutischen Bereiches mit Plasma spiegeln von 8 - 20 mg/l bereitet es außergewöhnliche galenische Schwierigkeiten, eine geeignete Zubereitung für die chronische orale Therapie zu schaffen, die im steady-state gleichmäßige, im therapeutischen Bereich liegende Blutspiegel, die auch in den Nachtstunden aufrechterhalten werden, gewährleistet. Besonders wünschenswert wäre im Hinblick auf die bei diesem Arzneistoff besonders wichtige Patienten-Compliance eine orale Darreichungsform, die es erlaubt, die gesamte Tagesdosis auf einmal einzunehmen.

In der DE-AS-2 336 218 ist eine für Theophyllin anwendbare Depot-Arzneiform mit linearer Wirkstofffreigabe beschrieben, bei der sphäroide Arzneistoffteilchen mit einer Dialysemembran überzogen sind, deren Filmbildner aus einem unlöslichen Celluloseether und einer Carboxylgruppen enthaltenden löslichen organischen Verbindung besteht. Mit dieser Depot-Arzneiform ist es möglich, eine lineare Abgabe von Theophyllin über 6 - 8 Stunden zu erreichen. Versucht man jedoch mit dieser Technik noch langsamere Abgaberaten zu erreichen, so weicht die Freisetzung beträchtlich von der Linearität ab.

Nach B. C. Lippold und H. Forster, Pharm. Ind. **44** (7), 735-740 (1982) erhält man durch Überziehen von Theophyllinpellets mit einem Lack aus Ethylcellulose und Polyethylenglykolen lineare, pH-unabhängige Abgaben von Theophyllin. Nach Angabe der Autoren ändert sich während der Lagerung die Freisetzungsrate der Pellets, so daß auch diese Darreichungsform weniger geeignet erscheint.

In der DE-OS-2 350 193 wird vorgeschlagen, auf wirkstoffhaltige Tablettenkerne mittels einer Tablettenpresse einen festen porösen Überzug aufzupressen. Eine verzögerte lineare Wirkstoffabgabe soll durch Variation der Dicke des Überzuges erreicht werden. Für eine Theophyllinzubereitung, die nur einmal am Tag verabreicht werden soll, ist dieses Verfahren nicht geeignet, weil wegen des benötigten großen Anteils an Hilfsstoffen die Tablette wegen ihres zu großen Volumens für den Patienten nicht mehr annehmbar ist.

Es sind zahlreiche sogenannte osmotische Freigabesysteme, beispielsweise aus der DE-PS-3 015 870, bekannt, die einen Wirkstoff gleichmäßig und weitgehend unabhängig von äußeren Einflüssen abgeben. Grundsätzlich sind jedoch Zubereitungen, die eine Tagesdosis Theophyllin über einen Zeitraum von bis zu etwa 16 Stunden kontinuierlich im resorptionsfähigen Teil des Gastrointestinaltraktes abgeben sollen, weniger geeignet, wenn sie als sogenannte "single-unit"-Dosisform vorliegen. Durch die enorme intra- und interindividuelle Variation der Magenentleerung ist nämlich der Durchgang der Zubereitung durch die für die Resorption wesentlichen Teile des Gastrointestinaltraktes weitgehend zufallsbedingt und nicht steuerbar. "Multiple-units"-Dosisformen, die aus genügend kleinen Untereinheiten bestehen, zeigen diese Abhängigkeit der Wirkstofffreigabe von der Magenentleerung bedeutend weniger, weil diese Untereinheiten den Pylorus auch dann passieren können, wenn der Schließmuskel geschlossen ist [H. Bedegaard, Acta Pharm. Technol. 28 (2), 149-157 (1982)].

Aus Barnes et al., New Engl. J. Med. **303**, 263-267 (1980) ist bekannt, daß beim Asthmakranken nachts zwischen 2 und 6 Uhr verschiedene Lungenfunktionsparameter, wie z. B. der Peak Expiratory Flow, die ungünstigsten Werte annehmen. Es erscheint daher wünschenswert, eine Theophyllin-Zubereitungsform zur Verfügung zu haben, die dem circadianen Verlauf der Krankheitssymptome angepaßte Theophyllin-Blutspiegelwerte liefert.

Eine solche Zubereitungsform sollte daher in den späten Nachtstunden die höchsten Theophyllinspiegel erzeugen. Dem steht erschwerend entgegen, daß während der Nacht die Aufnahme von Theophyllin aus dem Gastrointestinaltrakt verlangsamt ist.

Bei D. Nolte und H. Neumann, Therapiewoche 33, 1138-1141 (1983) wird über die Ergebnisse von Blutspiegeluntersuchungen nach täglich einmaliger Gabe eines auf den Markt befindlichen "Langzeittheophyllins" berichtet. Die Autoren stellen fest, daß diese Zubereitung zu betrachtlichen individuellen Schwankungen der Blutspiegelwerte führt. Durch diese Schwankungen besteht die Gefahr, daß ein Patient über weite Spannen des Tages subtherapeutische Theophyllin-Serumspiegel und während der Nacht Theophyllin-Serumspiegel aufweisen kann, die als im toxischen Bereich (mehr als 20 µg/ml) liegend zu betrachten sind.

Aufgabe der vorliegenden Erfindung ist die Zurverfügungstellung einer Theophyllin-Retardzubereitung, die bei einmaliger täglicher Einnahme möglichst unabhängig von den inter- und intraindividuell stark unterschiedlichen Passage- und Resorptionsbedingungen im Gastrointestinaltrakt Theophyllin-Plasmaspiegel erzeugt, die im therapeutischen Bereich liegen.

Gelöst wird diese Aufgabe durch eine Theophyllin-Retardzubereitung, die dadurch gekennzeichnet ist, daß sie Matrixpellets umfaßt, in denen Theophyllin-Teilchen eingebettet in einer Matrix aus wasserunlöslichem Kunststoff vorliegen, die mit einer Membran aus wasserunlöslichem Kunststoff mit eingebetteten Teilchen von Milchzucker umhüllt sind.

Die Matrixpellets werden zweckmäßigerweise durch Aufsprühen einer Suspension von feinkörnigem Theophyllin in einer Lösung eines wasserunlöslichen Kunststoffes in einem geeigneten organischen Lösungsmittel auf übliche inerte Tragerpellets hergestellt. Als Tragerpellets werden z.B. Zuckerkügelchen einer geeigneten Korngröße verwendet. Die Korngröße der Trägerpellets beträgt dabei etwa 0,2 bis 0,5, vorzugsweise 0,3 bis 0,4 mm. Für den Aufbau der Matrixpellets verwendet man einen physiologisch unbedenk-

2

lichen Kunststoff, der wasserunlöslich, jedoch in mindestens einem für galenische Zwecke geeigneten Lösungsmittel ausreichend löslich ist. Seine sonstigen Eigenschaften sind für die Zwecke der vorliegenden Erfindung wenig kritisch. Besonders geeignet als Kunststoff für die Matrixpellets ist Ethylcellulose.

Weitere gut geeignete Kunststoffe sind beispielsweise andere Celluloseether, Celluloseester, Polyvinylchlorid, Polyvinylalkohol und Acrylsäurepolymere. Es ist auch möglich, Gemische von Kunststoffen einzusetzen. Die Menge des bzw. der eingesetzten Kunststoffe beträgt etwa 2 bis 20 Gew.-% des Theophyllins, wobei ein Bereich von 5 bis 10 Gew.-% bevorzugt ist.

Als organische Lösungsmittel für die Herstellung der Matrixpellets sind solche zweckmäßig, die in der Galenik üblich sind und in denen Theophyllin unlöslich ist. Unter anderem sind die in der Galenik gebräuchlichen niederen Alkohole wie Ethanol und Isopropanol geeignet. Es können auch Gemische von miteinander mischbaren Lösungsmitteln verwendet werden, wie z. B. ein Gemisch von Ethanol und Isopropanol. Der Kunststoff, bzw. das Kunststoffgemisch wird in dem gewählten Lösungsmittel bzw. dem Lösungsmittelgemisch gelöst. In der entstandenen Lösung wird sodann feinkörniges Theophyllin suspendiert. Es ist zweckmäßig, Theophyllin einer Korngröße von unter 50 µm zu verwenden.

Die erhaltene Suspension wird auf an sich bekannte Trägerpellets, insbesondere Zuckerpellets, aufgetragen. Beispielsweise kann das Auftragen nach der dem Fachmann geläufigen Tauchrohrmethode erfolgen.

Besonders geeignet für die Zwecke der vorliegenden Erfindung sind Matrixpellets die Theophyllin in wässerigem Medium innerhalb einer Stunde möglichst vollständig freigeben, ohne daß die Matrixstruktur zerstört wird. Die zurückbleibenden Pellets sollten äußerlich nahezu unverändert aussehen und bei Betrachtung unter dem Mikroskop im Schnitt eine feinmaschige, netzartige Struktur aufweisen. Dem Fachmann ist es durch einfache versuche leicht möglich, durch Variation der verwendeten Ausgangsstoffe und deren Mengen sowie der Herstellungsparameter, Matrixpellets mit diesen Eigenschaften herzustellen.

Das Aufbringen der Membran auf die Matrixpellets erfolgt durch Aufsprühen einer Lösung eines Kunststoffs in einem nichtwässerigen Lösungsmittel, in der Milchzucker suspendiert ist.

Als Kunststoffe für den Aufbau der Membran sind solche geeignet, die wasserunlöslich sind, kein oder nur ein geringes Quellvermögen in Wasser besitzen, physiologisch verträglich sind und sich in Lösungsmitteln, wie sie in der Galenik üblich sind, genügend losen. Als Kunststoffe mit geringem Quellvermögen in Wasser werden für die Zwecke der vorliegenden Erfindung solche verstanden, die in wässerigem Medium nicht mehr als 5 Gew.-% Wasser aufnehmen. Als besonders geeignete Kunststoffe für die Membran werden Celluloseether und Celluloseester angesehen. Daneben eignen sich als Kunststoffe auch Polymere wie Polyvinylchlorid.

Milchzucker wird vorzugsweise in mikronisierter Form eingesetzt. Die Korngröße beträgt zweckmäßigerweise weniger als 20 µm, vorzugsweise weniger als 10 µm.

Das Verhältnis von Kunststoff zu Milchzucker kann in weiten Grenzen variiert werden. Bevorzugt ist ein Gewichtsverhältnis von Kunststoff zu Milchzucker von etwa 2 : 1 bis 1 : 3. Bevorzugt ist ein Gewichtsverhältnis von 4 : 3 bis 4 : 5.

Die Freisetzungsgeschwindigkeit des Theophyllin kann durch Variation der Zusammensetzung der Membran und/oder durch Variation der Schichtdicke der Membran in einem weiten Bereich gesteuert werden. So wird durch Erniedrigung der Schichtdicke der Membran, durch Erhöhung des Anteils an Milchzucker oder durch Verwendung des Milchzuckers in einer grobkörnigeren Form die Freigabegeschwindigkeit erhöht.

Das Auftragen der Membran auf die Matrixpellets erfolgt auf an sich bekannte Weise, insbesondere durch die verschiedenen Aufsprühtechniken. Hierzu wird eine Lösung des für die Membran vorgesehenen Kunststoffes bzw. Kunststoffgemisches in einem Lösungsmittel bzw. in einem Lösungsmittelgemisch bereitet und der mikronisierte Milchzucker vor dem Aufsprühen in dieser Lösung suspendiert. Nötigenfalls wird die Suspension während des Aufsprühens gerührt, um ein Absetzen des suspendierten Milchzuckers zu verhindern.

Die Membran kann die üblichen Hilfsstoffe, wie Weichmacher, Netzmittel und Farbstoffe enthalten. Geeignet sind pharmakologisch verträgliche Weichmacher z. B. aus der Reihe der Phthalsäure-, Phosphorsäure-, Zitronensäureester und Glyzerinester. Vorzugsweise wird Diethylphthalat verwendet. Netzmittel sind erforderlich, wenn der Überzug mit Farblacken eingefärbt werden soll. In Frage kommen z. B. Sorbitanfettsäureester oder Salze der Dioctylsulfobernsteinsäure.

Die neuen mit einer Membran versehenen Matrixpellets können zwar auch direkt, z. B. löffelweise, eingenommen werden, doch wird man eine dosierte Form im allgemeinen vorziehen.

Zweckmäßigerweise werden die mit einer Membran umhüllten Matrixpellets in Kapseln, vorzugsweise Gelatine-Steckkapseln, gefüllt. Zur Dosierung werden die Matrixpellets entweder abgewogen oder mit Dispensierscheren oder Abfüllvorrichtungen abgeteilt und mit Kapselfüllapparaten in die Kapseln gefüllt. Die umhüllten Matrixpellets können aber auch mit geeigneten Hilfsstoffen gemischt und zu Tabletten verpreßt werden. Durch die große mechanische Stabilität der Matrixpellets werden diese durch den Preßvorgang nicht beschädigt. Durch Wahl geeigneter Hilfsstoffe zerfällt eine solche Tablette nach dem Einnehmen in wenigen Minuten und gibt wie eine Kapsel die Matrixpellets frei.

Besonders vorteilhaft wird die der Erfindung zugrundeliegende Aufgabe gelöst durch Mischen von erfindungsgemäßen Matrixpellets mit unterschiedlicher Freigabecharakteristik.

Wie weiter oben ausgeführt, läßt sich durch Variation der Zusammensetzung und Schichtdicke der Membran die Freisetzungsgeschwindigkeit des Theophyllins in einem weiten Bereich steuern. Beispielsweise ist es möglich, eine 6-Stunden-Form (schnelle Form) und eine 12-Stunden-Form (langsame Form) herzustellen.

Unter einer 6- bzw. 12-Stundenform werden hierbei umhüllte Matrixpellets verstanden, die den Wirkstoff

3

EP 0 185 331 B1

über eine Zeit von 6 bzw. 12 Stunden im USP-Paddle-Modell (pH 7,4, Natriumphosphat-Puffer) stetig freisetzen.

Eine Zubereitung, worin 30 bis 70 Gew.-%, vorzugsweise 40 bis 60 Gew.-% und besonders bevorzugt 50 Gew.-% der Theophyllin-Tagesdosis als umhüllte Matrixpellets der schnellen Form (z. B. 6-Stundenform) und der Rest des Theophyllins als langsame Form (z. B. 12-Stundenform) vorliegt, ergibt bei der Verabreichung einen an den circadianen Verlauf des asthmatischen Krankheitsbildes optimal angepaßten Blutspiegelverlauf. Man erreicht hiermit, daß beispielsweise bei einer Einnahme einer Tagesdosis um etwa 19 Uhr der Theophyllin-Serumspiegel im steady-state in der Zeit von etwa 2 bis etwa 7 Uhr morgens ein Plateau erreicht, ohne in einen toxischen Bereich zu treten, und im weiteren Verlauf des Tages bis zur nächsten Einnahme weitgehend im therapeutischen Bereich verbleibt. Es hat sich außerdem gezeigt, daß überraschenderweise durch die Verwendung einer Mischung die an sich schon geringen inter- und intraindividuellen Serumspiegelfluktuationen nach wiederholter Gabe noch weiter reduziert werden. Theophyllin-Retardzubereitungen, die aus einem Gemisch von umhüllten Matrixpellets mit verschiedener Freisetzungscharakteristik bestehen, sind daher ein besonders bevorzugter Gegenstand der Erfindung.

Gewünschtenfalls kann die Plateauphase des Theophyllin-Blutspiegels während der Nacht verlängert werden, indem der Anteil an umhüllten Matrixpellets der langsamen Form erhöht wird.

Der Fachman ist somit in der Lage, durch Kombination einer "schnellen Form" mit einer "langsamen Form" eine nur einmal täglich einzunehmende Theophyllin-Retardzubereitung zur Verfügung zu stellen, die im steady-state dem circadianen Verlauf bronchokonstrikorischer Krankheitsbilder angepaße Blutspiegelwerte erzeugt.

Solche "gemischten" Theophyllin-Retardzubereitungen sind ein besonders bevorzugter Gegenstand der Erfindung.

Die erfindungsgemäßen mit einer Membran umhüllten Theophyllin-Matrixpellets zeigen eine weitgehend lineare Theophyllin-Freisetzung, die unabhängig von mechanischer Belastung, pH-Wert und Überflächenspannung des Testmediums erfolgt. Für die Produktion im technischen Maßstab ist von besonderer Bedeutung, daß die erfindungsgemäßen Matrixpellets eine überraschend hohe Chargenreproduzierbarkeit aufweisen und auch nach längerer Lagerzeit unter Stressbedingungen keine ins Gewicht fallende Änderung des Freigabeverhaltens zeigen.

Bei pharmakokinetischen Untersuchungen an Probanden zeigt sich, daß man durch Verabreichung der erfindungsgemässen Theophyllin-Retardzubereitung zu Blutspiegelwerten kommt, die nur eine sehr geringe, bei Zubereitungen nach dem Stand der Technik bisher nicht erreichte interindividuelle Streuung aufweisen. Hinzu kommt eine extrem kleine nach dem Stand der Technik bisher nicht erreichte peak-trough-Schwankung. Bei einer wiederholten Dosierung im 24-Stunden-Intervall beträgt der sogenannte swing als Maß für die Schwankung nur 50 % des nach dem Stand der Technik bisher erreichten. Mit der neuen Theophyllin-Retardzubereitung ist es möglich geworden, durch eine einmalige tägliche Dosierung im steady-state Theophyllin-Blutspiegelwerte zu erreichen, die gegenüber dem bisherigen Stand der Technik die Zeit im therapeutisch optimalen Bereich von 8 - 15 mg/l verdoppeln.

Weitere Gegenstände der Erfindung sind die in den Patentansprüchen gekennzeichneten Ausführungsformen sowie Verfahren zur Herstellung von erfindungsgemäßen Theophyllin-Retardzubereitungen. Die Verfahrensschritte in den erfindungsgemäßen Herstellungsverfahren sind dem Fachmann an sich bekannt.

## Herstellungsbeispiele

### 1. Matrixpellets

40 kg Ethylcellulose und 40 kg Polyvinylpyrrolidon werden in einer Mischung aus 800 l vergälltem Alkohol und 1200 l Isopropanol gelöst. In diese Lösung werden 800 kg Theophyllin (Korngröße < 50 µm) suspendiert. Diese Suspension wird auf 89 kg Zuckerpellets der Korngröße 0,3 - 0,4 mm aufgesprüht. Man erhält 969 kg Theophyllinpellets der Korngröße 0,9 - 1,1 mm und einem Theophyllingehalt von 82,5 %. Diese Matrixpellets geben Theophyllin in einem wässerigen Medium innerhalb 1 Stunde zu 100 % frei. Das zurückbleibende, äußerlich nahezu unveränderte Pellet besteht aus einem feinmaschigen, mit einem Auer-Glühstrumpf vergleichbaren Netzwerk aus Ethylcellulose.

Anstelle von Ethylcellulose können andere wasserunlösliche physiologisch inerte Polymere verwendet werden. Der Anteil der Polymeren kann zwischen 2 - 20 % des eingesetzten Theophyllins schwanken. Der bevorzugte Bereich beträgt zwischen 5 - 10 %. Besonders geeignete Polymere sind: Celluloseether, Celluloseester, Polyvinylchlorid, Polyvinylalkohol und Acrylsäurepolymere.

### 2. Umhüllen der Matrixpellets

#### 2.1. Ausgangsstoffe für eine Charge von 1,5 kg

| | |
|---|---|
| a) Theophyllin-Matrixpellets | 1417,50 g |
| b) Celluloseacetobutyrat CAB 381-05 | 37,50 g |
| c) Milchzucker mikronisiert | 41,25 g |
| d) Diethylphthalat | 3,75 g |
| e) Aceton | 350 ml |
| f) Isopropanol | 350 ml |

4

2.2. Ausführung

(b) wird in (e) gelöst. (c) wird ungefähr 3 Minuten mit einem wirbelnden Rührwerk (ULTRA-TURRAX® Typ T45) in (f) suspendiert. Die Lösung von (b) in (e) und die Suspension von (c) in (f) werden unter Rühren vereinigt. Anschließend wird (d) hinzugefügt.

In einem Wirbelschichtgranulator (Aeromatic STREA 1) wird die Suspension auf die Theophyllin-Matrixpellets aufgesprüht. Während des Sprühvorgangs wird die Suspension gerührt, um ein Absetzen des Milchzuckers zu verhindern. Nach dem Auftragen der Suspension werden die Matrixpellets ca. 30 Minuten bei einer Zulufttemperatur von etwa 60°C getrocknet.

2.3. Freigabeverhalten der umhüllten Matrixpellets

Das Freigabeverhalten von drei verschiedenen Chargen A, B und C wird in dem Paddle-Modell nach USP XX bei 100 Umdrehungen pro Minute bei einem pH-Wert von 7,4 (Puffer) bestimmt:

|  | Charge A | Charge B | Charge C |
|---|---|---|---|
| 1. Stunde | 7,3 % | 8,2 % | 7,6 % |
| 2. Stunde | 15,3 % | 16,1 % | 16,4 % |
| 3. Stunde | 23,8 % | 24,4 % | 25,5 % |
| 4. Stunde | 31,4 % | 32,6 % | 34,4 % |
| 5. Stunde | 39,0 % | 40,5 % | 43,0 % |
| 6. Stunde | 46,2 % | 48,3 % | 51,3 % |
| 7. Stunde | 53,6 % | 55,8 % | 58,8 % |
| 8. Stunde | 60,7 % | 63,1 % | 65,7 % |
| 9. Stunde | 67,2 % | 69,8 % | 71,9 % |
| 10. Stunde | 73,6 % | 76,0 % | 77,2 % |
| 11. Stunde | 79,9 % | 81,4 % | 81,6 % |
| 12. Stunde | 84,4 % | 86,1 % | 85,5 % |

Aus den gefundenen Freigabewerten ergibt sich, daß mit den erfindungsgemäßen Retardzubereitungen eine lineare Wirkstoffabgabe über einen langen Zeitraum verbunden mit einer sehr großen Chargenreproduzierbarkeit erzielbar ist.

3. <u>Steuerung des Freigabeverhaltens der umhüllten Mikropellets</u>

3.1. Verhältnis Milchzucker/Kunststoff 0,5 : 1

6 g Polyvinylalkohol und 24 g Ethylcellulose werden in einer Mischung aus 200 g Aceton und 200 g Isopropanol gelöst. In dieser Lösung werden 15 g mikronisierter Milchzucker einer durchschnittlichen Korngröße von 5 µm suspendiert.

In einem Wirbelschichtsprühgerät wird diese Suspension auf 955 g Theophyllin-Matrixpellets aufgesprüht.

Nach dem Aufsprühen von 1/3, 2/3 und 3/3 der Lösung wird je eine Probe zur Bestimmung der Theophyllinfreisetzung entnommen. Man erhält folgende Resultate:

| Stunden | Freigabe in % nach aufgetragener Überzugsmenge | | |
|---|---|---|---|
|  | 1/3 | 2/3 | 3/3 |
| 1 | 23,4 | 3,3 | 1,0 |
| 2 | 43,4 | 7,5 | 2,4 |
| 3 | 60,8 | 12,0 | 4,2 |
| 4 | 75,4 | 16,3 | 5,6 |
| 5 | 86,3 | 20,8 | 7,4 |
| 6 | 93,6 | 25,2 | 9,2 |
| 7 | 97,0 | 28,8 | 10,6 |

3.2. Verhältnis Milchzucker/Kunststoff 1 : 1

30 g Ethylcellulose und 4,5 g Triacetin werden in 400 g Ethylalkohol gelöst. 30 g mikronisierter Milchzucker werden in dieser Lösung suspendiert und auf 935,5 g Theophyllin-Matrixpellets in einem Wirbelschichtsprühgerät aufgesprüht. Nach dem Aufsprühen von 3/6, 4/6, 5/6 und 6/6 dieser Suspension wird eine Probe zur Bestimmung der Theophyllinabgabe entnommen. Man erhält folgendes Freigabeverhalten:

| Stunden | Freigabe in % nach aufgetragener Überzugsmenge | | | |
|---|---|---|---|---|
| | 3/6 | 4/6 | 5/6 | 6/6 |
| 1 | 37,5 | 22,8 | 12,5 | 11,4 |
| 2 | 68,1 | 45,6 | 27,0 | 25,0 |
| 3 | 87,8 | 65,7 | 41,8 | 39,0 |
| 4 | 96,6 | 81,6 | 56,4 | 51,9 |
| 5 | | 90,9 | 68,8 | 64,6 |
| 6 | | | 79,2 | 74,1 |
| 7 | | | 87,1 | 82,6 |
| 8 | | | 92,7 | 88,8 |

3.3 Verhältnis Milchzucker/Kunststoff 3 : 1

50 g Polyvinylchlorid werden in 200 g Aceton suspendiert und 200 g Tetrahydrofuran zugegeben. Man erhält eine klare Lösung. In 400 g Aceton werden 150 g mikronisierter Milchzucker einer durchschnittlichen Korngröße von 5 μm suspendiert. Polymerlösung und Milchzuckersuspension werden vereinigt und in einem Wirbelschichtsprühgerät auf 800 g Theophyllin-Matrixpellets aufgesprüht. Nach dem Aufsprühen von 4/10, 6/10, 7/10, 9110 und 10/10 dieser Suspension wird eine Probe zur Bestimmung der Theophyllinabgabe entnommen. Man erhält folgendes Freigabeverhalten:

| Stunden | Freigabe in % nach aufgetragener Überzugsmenge | | | | | |
|---|---|---|---|---|---|---|
| | 4/10 | 6/10 | 7/10 | 8/10 | 9/10 | 10/10 |
| 1 | 66,2 | 54,0 | 47,8 | 42,2 | 32,3 | 30,0 |
| 2 | 98,7 | 85,2 | 77,7 | 69,7 | 60,6 | 57,7 |
| 3 | 109,0 | 102,0 | 96,8 | 89,6 | 84,4 | 79,5 |
| 4 | | 109,0 | 105,2 | 101,4 | 99,1 | 96,2 |
| 5 | | | | 104,7 | 104,2 | 102,1 |

Wie die unter 3.1. bis 3.3. wiedergegebenen Herstellungsbeispiele zeigen, kann durch Variation der Schichtdicke der Umhüllung und/oder durch Änderung des Verhältnisses Milchzucker zu Kunststoff in der Umhüllung der Bereich der gewünschten Theophyllin-Freisetzung sehr weitgehend gesteuert werden.

4. <u>Freigabeverhalten in Abhängigkeit vom Prüfmedium</u>

Die nachfolgenden Versuche wurden mit einer erfindungsgemäßen Theophyllin-Retardzubereitung durchgeführt, bestehend aus Matrixpellets wie sie unter 1. beschrieben sind, die mit 3,3 Gew.-% einer Membran, bestehend aus Polyvinylalkohol, Ethylcellulose und Milchzucker im Gewichtsverhältnis 1 : 4 : 5 überzogen sind.

4.1. Freigabeverhalten in Abhängigkeit vom pH-Wert

Die nachstehende Tabelle gibt die Freisetzung von Theophyllin in Gew.-% im Eurand-Diffutester bei den pH-Werten 1,2, 6,5 und 7,4 wieder. Es sind Mittelwerte aus n = 6 Versuchen mit Standardabweichung angegeben.

| Zeit | n = 6 pH 1,2 | n = 6 pH 6,5 | n = 6 pH 7,4 |
|---|---|---|---|
| 1. Stunde | 9,5 ± 0,3 | 9,2 ± 0,4 | 9,7 ± 0,5 |
| 2. Stunde | 19,6 ± 0,6 | 17,8 ± 0,6 | 24,3 ± 1,0 |
| 3. Stunde | 30,3 ± 0,6 | 27,5 ± 0,8 | 34,0 ± 1,2 |
| 4. Stunde | 41,4 ± 0,9 | 36,3 ± 0,8 | 43,6 ± 1,5 |
| 5. Stunde | 51,3 ± 0,8 | 44,9 ± 1,0 | 54,4 ± 1,6 |
| 6. Stunde | 61,0 ± 1,0 | 53,3 ± 1,3 | 63,2 ± 1,4 |
| 7. Stunde | 69,7 ± 1,0 | 61,0 ± 1,7 | 71,4 ± 1,7 |
| 8. Stunde | 77,7 ± 1,1 | 67,8 ± 2,0 | 79,2 ± 1,5 |

4.2 Freigabeverhalten in Abhängigkeit von der Pufferkonzentration

Die nachstehende Tabelle gibt die Freisetzung von Theophyllin in Gew.-% im LSP XX Paddle-Gerät bei 100 Umdrehungen pro Minute in 0,2, 0,1 und 0,05 molarem Phosphatpuffer (pH 7,4) und in destilliertem Wasser

EP 0 185 331 B1

wieder. Es sind Mittelwerte aus n = 6 Versuchen mit Standardabweichung angegeben.

| Zeit | Phosphatpuffer pH 7,4 0,2 molar | Phosphatpuffer pH 7,4 0,1 molar | Phosphatpuffer pH 7,4 0,05 molar | dest. Wasser |
|---|---|---|---|---|
| 1. Stunde | 8,6 ± 0,3 | 9,8 ± 0,2 | 10,9 ± 0,3 | 9,9 ± 0,3 |
| 2. Stunde | 15,8 ± 0,4 | 19,4 ± 0,6 | 20,9 ± 0,8 | 20,1 ± 0,4 |
| 3. Stunde | 28,1 ± 0,4 | 28,8 ± 0,8 | 31,2 ± 0,6 | 30,1 ± 1,0 |
| 4. Stunde | 37,9 ± 0,6 | 39,1 ± 1,0 | 38,1 ± 1,0 | 40,4 ± 0,4 |
| 5. Stunde | 47,1 ± 0,7 | 48,9 ± 1,3 | 47,6 ± 1,0 | 49,7 ± 0,9 |
| 6. Stunde | 55,7 ± 0,8 | 58,5 ± 1,7 | 55,4 ± 1,2 | 59,5 ± 1,4 |
| 7. Stunde | 64,8 ± 0,9 | 68,3 ± 2,0 | 66,1 ± 1,1 | 68,5 ± 1,7 |
| 8. Stunde | 72,7 ± 0,8 | 76,6 ± 1,8 | 72,5 ± 0,6 | 76,6 ± 1,2 |

4.3. Freigabeverhalten in Abhängigkeit von der Überflächenspannung

Die nachstehende Tabelle gibt die Freisetzung von Theophyllin in Gew.-% im USP XX Paddle-Gerät bei 100 Umdrehungen pro Minute in 0,1 molarem Phosphatpuffer (pH 7,4) sowie im gleichen Medium unter Zusatz von 0,1 bzw. 1 % Tween 80 wieder.
Es sind Mittelwerte aus n = 6 Versuchen mit Standardabweichung angegeben.

| Zeit | Phosphatpuffer pH 7,4 0,1 molar | Phosphatpuffer pH 7,4 0,1 molar + 0,1 % Tween 80 | Phosphatpuffer pH 7,4 0,1 molar + 1 % Tween 80 |
|---|---|---|---|
| 1. Stunde | 9,8 ± 0,2 | 9,8 ± 0,3 | 11,5 ± 0,8 |
| 2. Stunde | 19,4 ± 0,6 | 19,0 ± 0,5 | 20,8 ± 1,0 |
| 3. Stunde | 28,8 ± 0,8 | 29,0 ± 0,7 | 29,2 ± 0,9 |
| 4. Stunde | 39,1 ± 1,0 | 39,5 ± 1,0 | 38,4 ± 1,1 |
| 5. Stunde | 48,9 ± 1,3 | 48,0 ± 1,2 | 48,2 ± 1,4 |
| 6. Stunde | 58,5 ± 1,7 | 56,4 ± 1,3 | 56,4 ± 1,5 |
| 7. Stunde | 68,3 ± 2,0 | 65,4 ± 1,3 | 65,8 ± 1,8 |
| 8. Stunde | 76,6 ± 1,8 | 73,4 ± 1,2 | 73,2 ± 1,6 |

Die Versuchsergebnisse nach 4.1. bis 4.3. zeigen, daß die erfindungsgemäßen umhüllten Theophyllin-Matrixpellets ein Freigabeverhalten zeigen, das überraschend wenig vom pH-Wert, von der Pufferkonzentration und der Überflächenspannung des Prüfmediums beeinflußt wird.

5. Theophyllin-Retardzubereitung mit an den circadianen Verlauf des asthmatischen Krankheitsbildes angepaßtem Freigabeverhalten

5.1. Langsame Form

Matrixpellets nach Beispiel 1 werden nach dem in Beispiel 2.2. angegebenen Verfahren mit einer Membran überzogen, wobei für eine Charge von 4,1 kg folgende Mengen an Ausgangsstoffen verwendet werden:

| | |
|---|---|
| a) Theophyllin-Matrixpellets | 3908,0 g |
| b) Celluloseacetatbutyrat (Cellit BP 300) | 92,0 g |
| c) Milchzucker mikronisiert | 92,0 g |
| d) Acetyltriethylcitrat | 9,2 g |
| e) Aceton | 920,0 ml |
| f) Isopropanol | 920,0 ml |

Die umhüllten Matrixpellets geben in dem Paddle-Modell nach USP XX bei 100 Umdrehungen pro Minute bei einem pH-Wert von 7,4 (Phosphatpuffer) den Wirkstoff stetig über einen Zeitraum von 12 Stunden frei (12-Stundenform).

5.2. Schnelle Form

Matrixpellets nach Beispiel 1 werden nach dem in Beispiel 2.2. angegebenen Verfahren mit einer Membran überzogen, wobei für eine Charge von 4,0 kg folgende Mengen an Ausgangsstoffen verwendet werden:

7

| | |
|---|---|
| a) Theophyllin-Matrixpellets | 3882,4 g |
| b) Celluloseacetatbutyrat (Cellit BP 300) | 56,0 g |
| c) Milchzucker mikronisiert | 56,0 g |
| d) Acetyltriethylcitrat | 5,6 g |
| e) Aceton | 560,0 ml |
| f) Isopropanol | 560,0 ml |

Die umhüllten Matrixpellets geben in dem Paddle-Modell nach USP XX bei 100 Umdrehungen pro Minute bei einem pH-Wert von 7,4 (Phosphatpuffer) den Wirkstoff stetig über einen Zeitraum von 6 Stunden frei (6-Stundenform).

### 5.3. Studie der Theophyllinserumspiegel

Es wird eine Theophyllinzubereitung hergestellt, die pro Dosierungseinheit 400 mg wasserfreies Theophyllin enthält, wovon 50 % als 12-Stundenform nach 5.1. und der Rest als 6-Stundenform (schnelle Form nach 5.2.1 vorliegen. Die Matrixpellets sind in einer Kapsel als Dosierungseinheit abgefüllt.

Es wurde eine Studie durchgeführt zur Bestimmung der mit dieser Zubereitung erreichbaren steady-state-Theophyllinserumspiegel im Vergleich zu dem derzeit in Deutschland einzigen auf dem Markt befindlichen Theophyllinpräparat, das einmal pro Tag zu dosieren ist. Der Vergleich wurde als randomisierte multiple-dose cross-over-Studie an gesunden männlichen Freiwilligen (Nichtraucher, 23 bis 33 Jahre alt, 69 - 80 kg schwer) angelegt. Es fanden zwei Behandlungsperioden von je sieben Tagen Dauer mit einer dazwischenliegenden Auswaschphase von ebenfalls sieben Tagen statt. In den Behandlungsperioden wurde jeweils die Tagesdosis von 800 mg Theophyllin abends um 7 Uhr in zwei Kapseln der erfindungsgemäßen Zubereitung bzw. in zwei Tabletten des Vergleichspräparates unter standardisierten Bedingungen verabreicht. Den Probanden wurden während jeder Behandlungsphase jeweils 36 Blutproben entnommen. Der Theophyllingehalt in den Proben wurde mittels HPLC (Doppelbestimmung) bestimmt.

Die Auswertung des Vergleichsversuches ergab, daß mit der erfindungsgemäßen Zubereitung (nachstehend A genannt) im Vergleich zu dem anerkannt guten Vergleichspräparat (nachstehend B genannt) folgende überraschende Vorteile erreicht werden:

a) Die Serumspiegel im steady-state schwanken bei A bedeutend weniger als bei B. A führt im Vergleich zu B zu einem im steady-state um 51 % verringerten Swing, wobei unter Swing die Differenz zwischen maximaler und minimaler Serumkonzentration in Relation zur minimalen Serumkonzentration verstanden wird. (Medianer prozentualer Swing bei A 167 %, bei B 337 %; maximale Konzentration $\pm$ Standardabweichung bei A 13,4 $\pm$ 2,8 mg/l, bei B 17,8 $\pm$ 4,3 mg/l.)

b) A führt im Vergleich zu B zu einer Verdoppelung der Plateauzeit im steady-state, d.h. der Zeit, in der die Theophyllinkonzentration um nicht mehr als 1 mg/l unter der maximalen Theophyllin-Konzentration liegt. (Mittlere Plateauzeit $\pm$ Standardabweichung bei A 5,5 $\pm$ 2,1 Stunden, bei B 2,7 $\pm$ 1,2 Stunden).

c) Der Zeitraum, währenddessen die Theophyllin-Serumkonzentration im steady-state innerhalb des als therapeutisch, vor allem für die späten Nachtstunden, wünschenswerten Bereichs von 8 bis 15 mg/l liegt, ist bei der Verabreichung von A um 50 % länger als bei der Verabreichung von B. (A: 14,2 $\pm$ 3,3 Stunden; B 9,6 $\pm$ 2,4 Stunden.)

d) A verursacht bei den Probanden erheblich weniger Nebenwirkungen. Dies wurde durch Erfassung der von den Probanden berichteten Nebenwirkungen ermittelt. Erfaßt wurden die für Theophyllin typischen Nebenwirkungen Einschlafstörungen, Schlafstörungen, Kopfweh, Herzklopfen, erhöhte Diurese, Konsistenzveränderungen des Stuhls, Tremor und Schwindel.

Für jeden Probanden wurden die Summen der Produkte aus der Häufigkeit der einzelnen Nebenwirkungen und der Schwere. (Skala von 0,5 bis 3) bei Gabe von A bzw. B ermittelt und über alle Probanden zu Maßzahlen für die einzelnen Nebenwirkungen aufsummiert.

Die Maßzahlen für Einschlaf- und Schlafstörungen erreichten bei A nur 65 bzw. 66 % der Werte von B. Kopfweh wurde bei Gabe von A auf 74 %, Herzklopfen auf 43 % und erhöhte Diurese auf 50 % reduziert. Die Nebenwirkung Stuhlveränderung erhöhte sich auf 138 %, während Tremor und Schwindel auf 33 % bzw. 19 % reduziert wurden.

## Patentansprüche

1. Theophyllin-Retardzubereitung, dadurch gekennzeichnet, daß sie Matrixpellets umfaßt, in denen Theophyllin-Teilchen eingebettet in einer Matrix aus wasserunlöslichem Kunststoff vorliegen, die mit einer Membran aus wasserunlöslichem Kunststoff mit eingebetteten Teilchen von Milchzucker umhüllt sind.

2. Theophyllin-Retardzubereitung nach Anspruch 1, dadurch gekennzeichnet, daß die Matrixpellets aus inerten Tragerpellets aufgebaut sind, auf die die Theophyllin enthaltende Matrix aufgetragen ist.

3. Theophyllin-Retardzubereitung nach Anspruch 2, dadurch gekennzeichnet, daß es sich bei den inerten Trägerpellets um Zuckerkügelchen handelt.

4. Theophyllin-Retardzubereitung nach Anspruch 1, dadurch gekennzeichnet, daß die Theophyllin-Teilchen eine Korngröße von weniger als 50 µm aufweisen.

5. Theophyllin-Retardzubereitung nach Anspruch 1, dadurch gekennzeichnet, daß der die Membran bildende wasserunlösliche Kunststoff in Wasser nicht quellbar ist.

6. Theophyllin-Retardzubereitung nach Anspruch 5, dadurch gekennzeichnet, daß es sich bei dem wasserunlöslichen Kunststoff um einen Celluloseether oder einen Celluloseester handelt.

7. Theophyllin-Retardzubereitung nach Anspruch 1, dadurch gekennzeichnet, daß die in die Membran eingebetteten Teilchen von Milchzucker eine Korngröße von weniger als 20 µm, vorzugsweise weniger als 10 µm aufweisen.

8. Theophyllin-Retardzubereitung nach Anspruch 1, dadurch gekennzeichnet, daß sie eine halbe Tagesdosis Theophyllin enthält.

9. Theophyllin-Retardzubereitung nach Anspruch 1, dadurch gekennzeichnet, daß als die Membran bildender Kunststoff ein solcher gewählt wird, in dem Milchzucker unlöslich ist.

10. Theophyllin-Retardzubereitung nach Anspruch 1, dadurch gekennzeichnet, daß ein Teil der Matrixpellets als langsame Form und ein Teil der Matrixpellets als schnelle Form vorliegt.

11. Theophyllin-Zubereitung nach Anspruch 10, dadurch gekennzeichnet, daß die Matrixpellets der langsamen Form mehr als 90 %, vorzugsweise mehr als 95 % des enthaltenen Theophyllin im Verlauf von 10 bis 14 Stunden, vorzugsweise 11 bis 13 Stunden, im USP-Paddle-Gerät bei pH 7,4 freisetzen und die Matrixpellets der schnellen Form mehr als 90 %, vorzugsweise mehr als 95 % des enthaltenen Theophyllin im Verlauf von 4 bis 8, vorzugsweise 5 bis 7 Stunden im USP-Paddle-Gerät bei pH 7,4 freisetzen.

12. Theophyllin-Zubereitung nach einem der Ansprüche 10 oder 11, dadurch gekennzeichnet, daß 30 bis 70, vorzugsweise 40 bis 60 % des Theophyllin als Matrixpellets der langsamen Form vorliegen.

13. Theophyllin-Zubereitung nach einem der Ansprüche 11 oder 12, dadurch gekennzeichnet, daß sie 50 % des Theophyllin als Matrixpellets enthält, die das enthaltene Theophyllin im Verlauf von 12 Stunden im USP-Paddle-Gerät bei pH 7,4 zu mehr als 95 % freisetzen und 50 % des Theophyllin als Matrixpellets enthält, die das enthaltende Theophyllin im Verlauf von 6 Stunden im USP-Paddle-Gerat bei pH 7,4 zu mehr als 95 % freisetzen.

14. Verfahren zur Herstellung einer Theophyllin-Retardzubereitung, dadurch gekennzeichnet, daß man mikronisiertes Theophyllin zusammen mit einem wasserunlöslichen Kunststoff zu Matrixpellets formt und diese mit einer Membran aus wasserunlöslichem Kunststoff mit eingebetteten Teilchen von Milchzucker überzieht.

15. Verfahren nach Anspruch 14, dadurch gekennzeichnet, daß man die Matrixpellets durch Aufsprühen einer Suspension von mikronisiertem Theophyllin in einer Lösung eines wasserunlöslichen Kunststoffes in einem organischen Lösungsmittel auf Trägerpellets herstellt.

16. Verfahren nach Anspruch 14, dadurch gekennzeichnet, daß das Überziehen der Matrixpellets durch Aufsprühen einer Suspension von Milchzucker in einer Lösung eines wasserunlöslichen Kunststoffs in einem organischen Lösungsmittel erfolgt.

17. Verfahren zur Herstellung einer Theophyllin-Retardzubereitung, dadurch gekennzeichnet, daß man nach einem der Ansprüche 14, 15 und 16 hergestellte, mit einer Dialysemembran überzogene Matrixpellets zu einer Dosierungseinheit zusammenfaßt.

18. Verfahren nach Anspruch 17, dadurch gekennzeichnet, daß Matrixpellets, die den Wirkstoff innerhalb von 10 bis 14 Stunden freigeben, mit Matrixpellets, die den Wirkstoff innerhalb von 4 bis 8 Stunden freigeben, zusammengefaßt werden.

## Claims

1. Theophylline sustained release formulation, which comprises matrix pellets in which theophylline particles are embedded in a matrix of water-insoluble plastic and which are enclosed by a membrane of water-insoluble plastic containing embedded particles of lactose.

2. Theophylline sustained release formulation as claimed in claim 1, wherein the matrix pellets are built up from inert carrier pellets onto which the matrix containing the theophylline is applied.

3. Theophylline sustained release formulation as claimed in claim 2, wherein the inert carrier pellets are sugar beads.

4. Theophylline sustained release formulation as claimed in claim 1, wherein the theophylline particles have a particle size of less than 50 µm.

5. Theophylline sustained release formulation as claimed in claim 1, wherein the water-insoluble plastic which forms the membrane is not swellable in water.

6. Theophylline sustained release formulation as claimed in claim 5, wherein the water-insoluble plastic is a cellulose ether or a cellulose ester.

7. Theophylline sustained release formulation as claimed in claim 1, wherein the particles of lactose embedded in the membrane have a particle size of less than 20 µm, preferably less than 10 µm.

8. Theophylline sustained release formulation as claimed in claim 1, which contains half a daily dose of theophylline.

9. Theophylline sustained release formulation as claimed in claim 1, wherein a plastic in which lactose is insoluble is chosen as the plastic which forms the membrane.

10. Theophylline sustained release formulation as claimed in claim 1, wherein some of the matrix pellets are in the slow form and some of the matrix pellets are in the rapid form.

11. Theophylline formulation as claimed in claim 10, wherein the matrix pellets of the slow form release more than 90 %, preferably more than 95 %, of the theophylline present in the course of 10 to 14 hours, preferably 11 to 13 hours, in a USP paddle apparatus at pH 7.4, and the matrix pellets of the rapid form release more than 90 %, preferably more than 95 %, of the theophylline present in the course of 4 to 8, preferably 5 to 7, hours in a USP paddle apparatus at pH 7.4.

12. Theophylline formulation as claimed in either of claims 10 or 11, wherein 30 to 70, preferably 40 to 60 % of the theophylline is in the form of matrix pellets of the slow form.

13. Theophylline formulation as claimed in either of claims 11 or 12, which contains 50 % of the theophylline as matrix pellets which release more than 95 % of the theophylline present in the course of 12 hours in a USP paddle apparatus at pH 7.4, and 50 % of the theophylline as matrix pellets which release more than 95 % of the theophylline present in the course of 6 hours in a USP paddle apparatus at pH 7.4.

14. Process for the preparation of a theophylline sustained release formulation, which comprises micronized theophylline being shaped to matrix pellets together with a water-insoluble plastic and these being coated with a membrane of water-insoluble plastic containing embedded particles of lactose.

15. Process as claimed in claim 14, wherein the matrix pellets are prepared by spraying a suspension of micronized theophylline in a solution of a water-insoluble plastic in an organic solvent onto carrier pellets.

16. Process as claimed in claim 14, wherein the matrix pellets are coated by spraying on a suspension of lactose in a solution of a water-insoluble plastic in an organic solvent.

17. Process for the preparation of a theophylline sustained release formulation, which comprises matrix pellets which have been prepared as claimed in any one of claims 14, 15 and 16 and coated with a dialysis membrane being combined to form a dosage unit.

18. Process as claimed in claim 17, wherein matrix pellets which release the active compound within 10 to 14 hours are combined with matrix pellets which release the active compound within 4 to 8 hours.

**Revendications**

1. Composition de théophylline-retard, caractérisée en ce qu'elle comprend des boulettes à matrice dans lesquelles des particules de théophylline sont présentes noyées dans une matrice en matière plastique insoluble dans l'eau, lesdites boulettes étant enrobées par une membrane en matière plastique insoluble dans l'eau dans laquelle sont incorporées des particules de lactose.

2. Composition de théophylline-retard selon la revendication 1, caractérisée en ce que les boulettes à matrice sont constituées par des granules de support inertes sur lesquels est déposée la matrice contenant la théophylline.

3. Composition de théophylline-retard selon la revendication 2, caractérisée en ce que ce qui concerne les granules de support inertes, sont des sphérules de sucre.

4. Composition de théophylline-retard selon la revendication 1, caractérisée en ce que les particules de théophylline présentent une granulométrie inférieure à 50 µm.

5. Composition de théophylline-retard selon la revendication 1, caractérisée en ce que la matière plastique insoluble dans l'eau formant la membrane n'est pas gonflable dans l'eau.

6. Composition de théophylline-retard selon la revendication 5, caractérisée en ce qu'en ce qui concerne la matière plastique insoluble dans l'eau, il s'agit d'un éther de cellulose ou d'un ester de cellulose.

7. Composition de théophylline-retard selon la revendication 1, caractérisée en ce que les particules de lactose incorporées dans la membrane présentent une granulométrie inférieure à 20 µm, de préférence inférieure à 10 µm.

8. Composition de théophylline-retard selon la revendication 1, caractérisée en ce qu'elle contient une demi-dose quotidienne de théophylline.

9. Composition de théophylline-retard selon la revendication 1, caractérisée en ce que comme matière plastique formant la membrane, est choisie une telle matière dans laquelle le lactose est insoluble.

10. Composition de théophylline-retard selon la revendication 1, caractérisée en ce qu'une partie des boulettes à matrice est présente en tant que forme lente et une partie des boulettes à matrice est présente en tant que forme rapide.

11. Composition de théophylline, selon la revendication 10, caractérisée en ce que les boulettes à matrice de la forme lente libèrent plus de 90 %, de préférence plus de 95 % de la théophylline contenue, dans l'intervalle de 10 à 14 heures, de préférence 11 à 13 heures, dans l'appareil à pales USP à pH 7,4 et que les boulettes à matrice de la forme rapide libèrent plus de 90 %, de préférence plus de 95 % de la théophylline contenue, dans l'intervalle de 4 à 8, de préférence 5 à 7 heures dans l'appareil à pales USP à pH 7,4.

12. Composition de théophylline selon l'une des revendications 10 ou 11, caractérisée en ce que 30 à 70, de préférence 40 à 60 % de la théophylline sont présents dans des boulettes à matrice de la forme lente.

13. Composition de théophylline selon l'une des revendications 11 ou 12, caractérisée en ce qu'elle contient 50 % de la théophylline dans des boulettes à matrice qui libèrent plus de 95 % la théophylline contenue, dans l'intervalle de 12 heures dans l'appareil à pales USP à pH 7,4 et qu'elle contient 50 % de la théophylline dans des boulettes à matrice qui libèrent plus de 95 % la théophylline contenue, dans l'intervalle de 6 heures dans l'appareil à pales USP à pH 7,4.

14. Procédé pour la préparation d'une composition de théophylline-retard, caractérisé en ce qu'on forme à partir de théophylline micronisée conjointement avec une matière plastique insoluble dans l'eau, des boulettes à matrice et que l'on revêt celles-ci avec une membrane en matière plastique insoluble dans l'eau dans laquelle sont incorporées des particules de lactose.

15. Procédé selon la revendication 14, caractérisé en ce que l'on prépare les boulettes à matrice par pulvérisation sur des granules de support, d'une suspension de théophylline micronisée dans une solution dans un solvant organique d'une matière plastique insoluble dans l'eau.

16. Procédé selon la revendication 1, caractérisé en ce que le revêtement des boulettes à matrice est réalisé par pulvérisation d'une suspension de lactose dans une solution dans un solvant organique d'une matière plastique insoluble dans l'eau.

17. Procédé de préparation d'une composition de théophylline-retard, caractérisé en ce que l'un groupe des boulettes à matrice revêtues d'une membrane de dialyse, préparées selon l'une des revendications 14, 15 et 16, en une dose unitaire d'administration.

18. Procédé selon la revendication 17, caractérisé en ce que l'on groupe des boulettes à matrice qui libèrent la substance active en 10 à 14 heures, avec des boulettes à matrice qui libèrent la substance active en 4 à 8 heures.